Europäisches Patentamt

European Patent Office

Office européen des brevets

(19)

(11) Veröffentlichungsnummer: **0 331 068 B1**

(12) **EUROPÄISCHE PATENTSCHRIFT**

(45) Veröffentlichungstag der Patentschrift: **04.11.92**

(21) Anmeldenummer: **89103388.8**

(22) Anmeldetag: **27.02.89**

Die Akte enthält technische Angaben, die nach
dem Eingang der Anmeldung eingereicht wurden
und die nicht in dieser Patentschrift enthalten sind.

(51) Int. Cl.5: **G01N 33/53**, G01N 33/563,
G01N 33/577, G01N 33/543,
//G01N33/574,G01N33/74

(54) Immunoassay und -rolymere IgG-Derivate zur Kompensation von Störfaktoren in Immunoassays.

(30) Priorität: **03.03.88 US 164054**

(43) Veröffentlichungstag der Anmeldung:
**06.09.89 Patentblatt 89/36**

(45) Bekanntmachung des Hinweises auf die
Patenterteilung:
**04.11.92 Patentblatt 92/45**

(84) Benannte Vertragsstaaten:
**AT BE CH DE ES FR GB GR IT LI LU NL SE**

(56) Entgegenhaltungen:
**EP-A- 0 083 869**
**EP-A- 0 163 312**

**CLINICAL CHEMISTRY, Band 32, Nr. 8, 1986;
L.M.BOSCATO et al., Seiten 1491-1495**

**PATENT ABSTRACTS OF JAPAN, Band 8, Nr.
219 (P-306)[1656], 05 Oktober 1984**

(73) Patentinhaber: **BOEHRINGER MANNHEIM
GMBH
Sandhofer Strasse 116
W-6800 Mannheim 31(DE)**

(72) Erfinder: **Lenz, Helmut, Dr.rer.nat.
v. Kühlmann-Str. 14
W-8132 Tutzing(DE)**
Erfinder: **Mössner, Ellen, Dr.rer.nat.
Hallberger Allee 8
W-8132 Tutzing(DE)**
Erfinder: **Stock, Werner, Dr.rer.nat.
Grawolfstr. 2
W-8032 Gräfeling(DE)**
Erfinder: **Franken, Norbert, Dr.rer.nat.
Wilhelm Mayr-Str. 11
W-8000 München 21(DE)**
Erfinder: **McCarthy, Robert Crance, Dr.
12103 Brookshire Parkway
Carmel, IN 46032(US)**
Erfinder: **Röder, Albert, Dr.rer.nat.
Nördl. Seestr. 12
W-8193 Ammerland/Münsing(DE)**
Erfinder: **Haug, Harald, Dr.rer.nat.
Kreuzeckerstr. 12
W-8123 Peissenberg(DE)**

EP 0 331 068 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein verbessertes Verfahren zur Bestimmung einer immunologisch bindefähigen polyvalenten Substanz in Gegenwart von mindestens zwei für die immunologisch bindefähige Substanz spezifischen Reaktanten und in Gegenwart eines Inhibitors zur Kompensation von Störfaktoren in menschlichen Serumproben sowie ein hierfür geeignetes Reagenz.

Die empfindliche Bestimmung von immunologisch bindefähigen Substanzen, wie beispielsweise von polyvalenten Antigenen (Peptide, Proteine, Polysaccharide, Viren, Bakterien, spezifische Zellen) unter Verwendung von zwei oder gegebenenfalls mehreren Antikörpern, die gegen räumlich verschiedene Antigendeterminanten gerichtet sind, ist bekannt als immunoradiometrischer bzw. immunoenzymometrischer Sandwichassay (two-site-immunoassay). Am gebräuchlichsten ist die Durchführung dieses bekannten Bestimmungsverfahrens, wobei das zu bestimmende Antigen (Probe) mit einem ersten Antikörper, der entweder in fester Phase an ein geeignetes Trägermaterial wie Sepharose, Agarose, Kuntststoffröhrchen und dergleichen gebunden ist oder homogen, wie beispielsweise biotinyliert in Lösung vorliegt, und einer bestimmten Menge eines zweiten oder weiteren markierten Antikörpern in flüssiger Phase inkubiert wird. Die Spezifität des zweiten und gegebenenfalls weiterer Antikörper wird bevorzugt so gewählt, daß sie gegen andere Determinanten des zu bestimmenden Antigens als der erste Antikörper gerichtet sind, um eine Konkurrenz zwischen den Antikörpern um dieselben Bindungsstellen an dem zu bestimmenden Antigen auszuschließen, da hierdurch die Testempfindlichkeit beeinträchtigt würde. Sowohl der erste, festphasengebundene bzw. biotinylierte als auch der zweite bzw. weitere in Lösung vorhandene, markierte Antikörper werden im Überschuß zugesetzt. Das jeweilige Antigen kann entweder über die an den ersten Antikörper fixierte oder die in Lösung verbliebene, nicht immunologisch gebundene Aktivität bestimmt werden. Im letzteren Fall ist es erforderlich, eine definierte Menge von markierten zweiten Antikörper zuzugeben.

Aufgrund der erforderlichen Spezifitäten werden für die immunologischen Sandwichassays insbesondere von monoklonalen Antikörpern (MAKs) abgeleitete vollständige IgGs bzw. deren immunologisch reaktive Fragmente (Fab, F(ab$'$)$_2$) verwendet. Es ist jedoch zudem möglich, immunreaktive Bestandteile in diese Teste einzusetzen, die sich von polyklonalen Antikörpern (PAKs) ableiten.

Obwohl in den beschriebenen Two-Site-Immunoassays für den zu bestimmenden Analyten spezifische Antikörper verwendet werden, treten mit signifikanter Häufigkeit Humanserumproben auf, die unspezifische Reaktionen hervorrufen. Diese führen zu falschen Testresultaten mit entsprechend gravierenden Konsequenzen für die therapeutischen Maßnahmen. Das Auftreten von unspezifischen Reaktionen ist auf in der Probe vorhandene Substanzen zurückzuführen, die gleichfalls wie der zu bestimmende Analyt (polyvalentes Antigen) die spezifischen Immunglobulinreagentien binden (Störfaktoren), jedoch an anderen Angriffspunkten.

Üblicherweise werden daher solchen Immunoassays präventiv im Überschuß unspezifische Immunglobuline oder Immunglobulinfragmente (in der Regel handelt es sich hierbei um Immunglobulin G, IgG) von derselben Tierspezies, von der die spezifischen Antikörper stammen, zugesetzt (G. M. Addison in Radioimmunoassay and Related Procedures in Medicine, Vol. 1, 131 - 147 (1974); EP-A 0174 026). Seit der Verwendung von spezifischen monoklonalen Antikörpern, welche in der Regel von der Maus stammen, in Immunoassays erfordert die genannte Entstörmaßnahme große Mengen von nicht spezifischen Maus-IgG in Form von Maus-Serum, Maus-Ascites oder isoliertem Maus-Immunglobulin (ca. 300 - 500 ug/ml; Clin. Chem. 32, 1491 - 1495 (1986) ). Beispielsweise wird nach EP-A 0174 026 durch Zusatz von ca. 30 ug/ml relevantem, nicht spezifischem Maus-bzw. Ratten-IgG zu Immunoassays der geschilderten Art nur bei bestimmten Seren und in besonderen Fällen (negative Proben) eine vollständige Kompensation von Störungen erreicht. Die Bereitstellung von Maus-IgG im erforderlichen Kilomaßstab ist jedoch mit den derzeit verfügbaren Methoden zu wirtschaftlich interessanten Bedingungen nicht möglich und zudem aus ethischen Gesichtspunkten kritisch.

Eine wesentliche Maßnahme zur Vermeidung von Störungen in Immunoassays der genannten Art ist die Verwendung von Fab-bzw. F(ab')$_2$-Fragmenten für mindestens einen der in dem Immunoassay verwendeten spezifischen Antikörper. Hiermit werden alle Störfaktoren in der Probe, die auf Fc-Teile von IgG gerichtet sind (Rheumafaktoren, Anti-Fc-Immunglobuline wie z. B. IgM), ihres Angriffspunkts auf einem der spezifischen Immunreagentien beraubt und brauchen somit nicht kompensiert zu werden.

Jedoch treten bei manchen Humanseren in Immunoassays trotz der verwendeten Fc-freien spezifischen Antikörperreagentien weiterhin Störungen auf. Diese sind auf Substanzen im Serum zurückzuführen, die auf Fab bzw. F(ab')$_2$ gerichtet sind. Nach EP-B 0083 869 erkennen solche Störfaktoren Fab-Bereiche jedoch nur, wenn diese vom Fc-Teil abgetrennt sind. Diese lassen sich bei entsprechenden Immunoassays durch den Zusatz von nativen oder vernetzten, für das zu bestimmende Antigen nicht spezifischen Fab- bzw. F-

(ab')$_2$-Fragmenten beseitigen (EP-B 0083 869). Dabei zeigen aggregierte Fab- bzw. F(ab')$_2$-Fragmente im Vergleich zu den nativen Bestandteilen eine 2 - 3fach höhere Entstörwirkung. Vollständige nicht spezifische IgGs bewirken nach EP-B 0083 869 dagegen sowohl in nativer als auch in aggregierter Form in den genannten Immunoassays keine Entstörung. Das Verfahren hat zudem den wesentlichen Nachteil, daß hierbei - neben den für das zu bestimmende Antigen spezifischen Immunglobulinreagentien - beträchtliche Mengen hochwertiger nicht spezifischer Immunglobulinreagentien benötigt werden, was erhebliche wirtschaftliche sowie ethische Probleme mit sich bringt. Danach werden beispielsweise zur Entstörung mindestens 100 ug/ml der sich als wirkungsvoller erwiesenen aggregierten nicht spezifischen Fc-freien Immunglobulinfragmente eingesetzt.

Der Erfindung liegt daher die Aufgabe zugrunde, neue Möglichkeiten für die Kompensation von unspezifischen Reaktionen bei Immunoassays mit Fc-haltigen und Fc-freien spezifischen Antikörperreagentien als spezifische Reaktanten zur Verfügung zu stellen, wodurch auf vollständige IgGs und auf Fab- bzw. F(ab')$_2$-Fragmente gerichtete Störfaktoren in Humanseren verläßlich beseitigt werden, und unter Berücksichtigung ethischer Gesichtspunkte eine wirtschaftliche Nutzung der verbesserten Testsysteme erlauben.

Diese Aufgabe wird erfindungsgemäß gelöst durch ein Verfahren zur Bestimmung einer immunologisch bindefähigen Substanz in einer biologischen Flüssigkeit von einer ersten Tierart oder vom Menschen in Gegenwart von mindestens zwei für die immunologisch bindefähige Substanz spezifischen Reaktanten einer zweiten Tierart, von denen mindestens einer ein Fc-haltiges Antikörperreagenz ist, und in Gegenwart eines Inhibitors zur Kompensation von Störfaktoren, dadurch gekennzeichnet, daß das Reaktionsgemisch mit 0,1 - 50 $\mu$g/ml für die immunologisch bindefähige Substanz nicht spezifischen, von monoklonalen oder polyklonalen Antikörpern abgeleiteten, nichtpartikulären Aggregaten einer zweiten und/oder dritten Tierart zur Kompensation von Störfaktoren in Berührung gebracht wird.

Als immunologisch bindefähige Substanzen kommen insbesondere polyvalente Antigene wie Peptide, Proteine, Polysaccharide, Viren, Bakterien und andere spezifische Zellen bzw. Bestandteile von diesen in Betracht. Als spezifische Reaktanten für die jeweils zu bestimmende immunologisch bindefähige Substanz konnen sowohl polyklonale als auch monoklonale Antikörper eingesetzt werden, bevorzugt werden hierbei IgGs und deren immunreaktiven Bestandteile wie Fab- bzw. F(ab')$_2$-Fragmente kombiniert verwendet. Besonders bevorzugt ist der Einsatz von zwei oder gegebenenfalls mehreren spezifischen Reaktanten, von denen mindestens einer ein Fab- bzw. F(ab')$_2$-Fragment und die übrigen spezifischen Reaktanten vollständige IgGs sind.

Als für die immunologisch bindefähige Substanz nicht spezifische Antikörper-Aggregate zur Kompensation von Störungen werden bevorzugt Aggregate von für die immunologisch bindefähige Substanz nicht spezifischen IgGs verwendet, besonders bevorzugt ein IgG-Aggregat bestehend aus unspezifischem IgG und einem weiteren Makromolekül. Bevorzugt sind hierbei nicht spezifische IgGs, die von derselben Tierspezies wie mindestens einer der spezifischen Reaktanten stammen. Als Makromoleküle sind hier beispielsweise Fab- bzw. F(ab')$_2$-Fragmente, d. h. Fc-freie IgG-Fragmente, die von der Maus oder einer anderen Spezies sich ableitenden MAKS oder PAKs stammen können, sowie Proteine (z. B. Albumin), Polysaccharide (z. B. Dextran) oder andere wasserlösliche Polymere geeignet. Außerdem können Heteropolymere, welche beispielsweise aus Maus-IgG über verbrückendes Rinder-IgG gebildet werden, zur Kompensation von Störungen verwendet werden. Besonders bevorzugt werden Aggregate bestehend aus einem IgG-Molekül und einem Fc-freien IgG-Fragment, wobei sowohl die IgGs als auch die Fc-freien IgG-Fragmente aus derselben Tierspezies wie einer der spezifischen Reaktanten stammen, verwendet. Solche IgG/Fab- bzw. F(ab')$_2$-Polymere zeigen sogar eine um den Faktor ca. 3 bessere Entstörwirkung als reine IgG-Aggregate. Abhängig von der individuellen Serumprobe sind 0,1 - 50 $\mu$g/ml eines IgG-Mischpolymeren für eine erfolgreiche Enstörung ausreichend, bevorzugt wird jedoch mit Konzentrationen von 5 bis 25 $\mu$g/ml und besonders bevorzugt mit Konzentrationen von 5 bis maximal 10 $\mu$g/ml gearbeitet, da in den meisten Fällen schon ein weitaus geringerer Zusatz unspezifischer IgG-Aggregate voll wirksam ist.

Gemäß einer bevorzugten Ausführungsform der Erfindung werden zur Kompensation von Störfaktoren in Humanseren unspezifische homopolymere oder heteropolymere IgG-Aggregate oder IgG/Fab-bzw. F-(ab')$_2$-Aggregate mit Molekulargewichten von 320.000 Dalton und mehr eingesetzt. Dabei enthalten die IgG-Aggregate IgG von der Spezies, aus der mindestens einer der spezifischen Reaktanten stammt und gehören bevorzugt derselben Subklasse wie mindestens einer der spezifischen Reaktanten an. Bevorzugt werden hierbei polymere IgG-Präparate mit Molekulargewichten von 320.000 bis 10 Millionen Dalton verwendet, wobei die Kompensationswirkung gegenüber Serumstörfaktoren mit steigendem Molekulargewicht wächst. Besonders bevorzugte Ausführungsformen der Erfindung sind Two-Site-Immunoassays mit zwei oder mehreren spezifischen Maus- bzw. Ratten-MAK-Reaktanten, von denen mindestens ein Reaktant ein Fab- oder F(ab')$_2$-Fragment und mindestens einer der anderen Reaktanten ein Fc-haltiges IgG ist, bei denen zur Kompensation von Störfaktoren homopolymere oder heteropolymere nicht spezifische IgG-Aggregate oder IgG/Fab- bzw. F(ab′)₂-Aggregate mit Molekulargewichten größer als 320.000 Dalton zugesetzt werden, wobei die in den unspezifischen Aggregaten enthaltenen IgGs und Fab- bzw. F(ab′)₂-Fragmente monoklonal und von der Spezies und Subklasse sind wie mindestens einer der spezifischen Reaktanten. Dabei ist gleichgültig, ob das nicht spezifische IgG bzw. Fab-Fragment über Ascites, Fermentation oder über Genetic Engineering durch Expression in Mikroorganismen hergestellt wird.

Die nach dem erfindungsgemäßen Verfahren für die Kompensation von Störungen geeigneten polymerisierten unspezifischen IgG-Moleküle bzw. die unspezifischen IgG-/Fab- bzw. F(ab′)₂-Aggregate müssen sich von einer anderen Spezies, als von der die zu analysierende Probe stammt, ableiten. In der Regel werden immunologische Bestimmungsverfahren für die Analyse von Humanseren mit Hilfe von spezifischen, von der Maus sich ableitenden monoklonalen Antikörpern bzw. deren immunreaktiven Fragmenten verwendet. Die spezifischen Antikörperreagentien können jedoch auch von einer anderen Tierspezies stammen. Die zur Enstörung zugesetzten unspezifischen Antikörperaggregate können entsprechend von der Maus oder in Kombination mit Maus-IgG bzw. deren Fab-Fragmente von einer anderen, von der ersten unterschiedlichen Tierspezies stammen. Beispielsweise können Maus-IgG/Rinder-IgG-Heteropolymere (s.o.) zur Enstörung bei Immunoassays, die als spezifisches Antikörperreagenz Maus-MAKs bzw. Maus-IgGs verwenden, eingesetzt werden.

Durch den erfindungsgemäßen Zusatz unspezifischer IgG-Aggregate bzw. IgG/Fab- bzw. F(ab′)₂-Aggregate wird im Vergleich zu nativen IgGs eine um den Faktor 20 - 1000 und im Vergleich zu nativen oder aggregierten Fc-freien IgG-Fragmenten eine um den Faktor 20 - 1500 bzw. um das 3fache gesteigerte Wirksamkeit für die Kompensation von unspezifischen Reaktionen bei Immunoassays mit mindestens zwei für das zu bestimmende Immunogen spezifischen Reaktanten, von denen sich mindestens einer von einem Fab- bzw. einem F(ab′)₂-Fragment ableitet, erreicht. Dies ist überraschend, da nicht vorauszusehen war, daß IgG-haltige unspezifische Reaktanten bei Immunoassays, an denen Fc-freie spezifische Immunglobulinreagentien von monoklonalen oder polyklonalen Antikörpern beteiligt sind, überhaupt eine Kompensation von auf Fab- bzw. F(ab′)₂-Fragmente gerichtete Störungen bewirken. Des weiteren führt EP-B 0083869 als nächstliegender Stand der Technik den Fachmann eher zu einer gegenteiligen Annahme. Denn in dieser Patentschrift wird ausdrücklich betont, daß sowohl native als auch aggregierte IgGs keinen entstörenden Effekt bei dem dort genannten immunologischen Agglutinationstest zeigen und die zur Enstörung wie auch die für die spezifische Agglutination zugesetzten Agenzien IgG-frei sein müssen.

Unspezifisches IgG kann mit sich selbst oder mit Antikörper-Fragmenten sowie mit Proteinen, Polysacchariden oder anderen wasserlöslichen Makromolekülen durch Hitzeeinwirkung, chemisch oder nicht kovalent über bioaffine Wechselwirkung nach aus der Literatur bekannten Methoden vernetzt werden. In jedem Fall entstehen in wässrigen Pufferlösungen lösliche Aggregate. Die chemische Vernetzung kann beispielsweise durch homo- und heterobifunktionelle chemische Verbindungsarme (Linker), über Proteine oder aktiviertes Dextran oder durch Selbstvernetzung der IgG-Moleküle bzw. ihrer und/oder anderer Fc-freien Fragmente mit Carbodiimid erfolgen. Außerdem ist es möglich, die Antikörpermonomeren über Disulfidreduktion und Reoxidation oder über Oxidation ihres Kohlenhydratanteils mit sich selbst oder einem geeigneten Makromolekül zu vernetzen. Als chemische Linker kommen beispielsweise Bis(maleinimido)-methylester, Dimethyl-suberimidat, Disuccinimidyl-suberat, Glutardialdehyd, N-Succinimidyl-3-(2-pyridyldithio)propionat, N-5-Azido-2-nitrobenzoylsuccinimid, N-Succinimidyl(4-Iodacetyl)-aminobenzoat oder die Kombination von Maleinimidohexanoylsuccinimidat und S-Acetyl-mercaptobernsteinsäureanhydrid bzw. analoger Verbindungen in Betracht. Die Herstellung von aktiviertem Dextran kann beispielsweise aus Aminodextran eines definierten Molekulargewichts mit Maleinimidohexanoylsuccinimidat erfolgen, welches anschließend mit Mercaptoacetyl-derivatisiertem unspezifischen IgG-Molekülen vernetzt wird. Mit bioaffiner Wechselwirkung sind allgemein solche Bindungen gemeint, wie sie z. B. bei den Paaren Biotin/Avidin (bzw. Streptavidin), Hapten/anti-Hapten-Antikörper, Antigen/anti-Antigen-Antikörper, Ligand/Bindeprotein (z. B. Thyroxin/Thyroxinbindendes Protein), Hormon/Rezeptor vorliegen. Eine geeignete Ausführungsform ergibt sich durch mindestens zweifache Biotinylierung des unspezifischen IgG und Zugabe von Streptavidin oder durch Derivatisierung des IgG mit mindestens zwei Digoxigeninmolekülen und Vernetzung mit einem monoklonalen oder polyklonalen anti-Digoxin-Antikörper. Ebenfalls geeignet sind Aggregate, die durch Umsetzung von Poly-Humanalbumin mit einem monoklonalen anti-Humanalbumin-Antikörper entstehen.

Die jeweils erhaltenen Aggregat-Rohgemische können nach Dialyse direkt verwendet, oder beispielsweise durch Gelfiltration in Fraktionen mit steigendem Molekulargewicht aufgetrennt werden und anschließend direkt zur Kompensation von Störfaktoren in Immunoassays eingesetzt werden.

Einer der dem Testgemisch zugesetzten spezifischen Reaktanten, vorzugsweise der Fc-haltige IgG-Antikörper, wird in dem Fachmann bekannter Weise in fester Phase an ein Trägermaterial wie beispielsweise Agarose oder Kuntststoffröhrchen und dergleichen gebunden oder liegt - beispielsweise verbunden mit Biotin - in flüssiger Phase vor. Wird ein biotinylierter erster Antikörper verwendet, wird zunächst der Komplex aus dem Antigen und einem markierten zweiten spezifischen Reaktanten gebildet, der dann in situ an ein mit einem Biotin-bindenden Protein, wie beispielsweise Avidin oder Streptavidin beschichtetes Trägermaterial gebunden wird. Daneben sind jedoch auch andere Testführungen mit erstem biotinyliertem Antikörper möglich: Das Antigen reagiert mit dem biotinylierten MAK und wird in situ oder nach einer bestimmten Vorinkubationsperiode an eine Biotin-bindende Festphase gebunden und dann erst mit dem zweiten spezifischen Reaktanten zusammengebracht. Oder es ist möglich, daß zunächst das Antigen mit dem zweiten spezifischen Reaktanten und anschließend mit dem biotinylierten Antikörper reagiert. Die Markierung des zweiten und gegebenenfalls weiterer spezifischer Reaktanten, bei denen es sich vorzugsweise um Fc-freie IgG-Fragmente handelt, erfolgt durch Kupplung mit einem Enzym, einer fluoreszierenden, chemiluminezierenden oder radioaktiven Substanz. Verfahren zur Markierung von derartigen Antikörperderivaten sind dem Fachmann bekannt, beispielsweise aus E. Ischikawa et al., J. of Immunoassay 4 (1983) 209 - 327, und bedürfen hier keiner weiteren Erläuterung.

Ein weiterer Gegenstand der Erfindung sind diagnostische Mittel zur Bestimmung von immunologisch bindefähigen polyvalenten Substanzen in Two-Site-Immunoassays, welche die beschriebenen unspezifischen Antikörper-Aggregate enthalten.

Das diagnostische Mittel enthält neben zwei oder gegebenenfalls mehreren spezifischen Antikörpern, von denen einer ein IgG-Molekül und mindestens einer ein Fc-freies IgG-Fragment ist, ein geeignetes Puffersystem und die beschriebenen unspezifischen Antikörper-Aggregate sowie gegebenenfalls weitere üblicherweise verwendete Hilfsstoffe, wie beispielsweise Reaktionsbeschleuniger, Detergentien oder Stabilisatoren. Als geeignete Puffersysteme können beispielsweise 20 - 60 mM Phosphatpuffer (pH 7.0) oder ein 50 mM HEPES/100 mM NaCl-Puffersystem (pH 7.4) verwendet werden. Als Reaktionsbeschleuniger kommen hier beispielsweise Dextransulfat oder Polyethylenglykol 6000 bis 40 000, als Detergentien z. B. Triton® x-100, Tween® 20 oder Pluronic® F 68 und als geeignete Stabilisatoren Phenol, Oxypyrion, Chloracetamid, Merthiolat u. a. in Betracht.

Das diagnostische Mittel enthält die unspezifischen Antikörper-Aggregate mit Molekulargewichten von 320 000 Dalton und mehr, vorzugsweise bis 10 Millionen Dalton, in einer Konzentration von 0,1 bis 50 $\mu$g/ml, vorzugsweise in einer Konzentration von 5 bis 25 $\mu$g/ml und besonders bevorzugt in einer Konzentration von 5 bis 10 $\mu$g/ml.

Das diagnostische Mittel kann in Form einer Lösung oder eines Trockenchemiereagenzes auf einen saugfähigen Träger aufgezogen oder in einem offenen Film vorliegen.

Das erfindungsgemäße diagnostische Mittel in Form einer Lösung, gegebenenfalls auf den gewünschten pH-Wert gepuffert, enthält vorzugsweise sämtliche für den Test benötigten Reagentien. Aus Haltbarkeitsgründen kann es vorteilhaft sein, die für den Test benötigten Reagentien auf zwei oder mehr Lösungen zu verteilen, die erst bei der eigentlichen Untersuchung vermischt werden. Dabei ist unerheblich, ob die unspezifischen Antikörper-Aggregate getrennt in einem geeigneten Puffersystem oder/und mit einem oder/und beiden bzw. weiteren spezifischen Antikörpern vorgelegt werden.

Zur Herstellung des diagnostischen Mittels in Form eines Teststreifens wird ein saugfähiger Träger, vorzugsweise Filterpapier, Cellulose oder Kunstfaservlies mit Lösungen der erforderlichen, üblicherweise zur Herstellung von Teststreifen verwendeten Reagenzien in leicht flüchtigen Lösungsmitteln, wie z. B. Wasser, Methanol, Ethanol oder Aceton imprägniert. Dies kann in einem Imprägnierungsschritt erfolgen. Oft ist es jedoch zweckmäßig, die Imprägnierung in mehreren Schritten durchzuführen, wobei Lösungen eingesetzt werden, die jeweils einen Teil der Bestandteile des diagnostischen Mittels enthalten.

Zur Herstellung des diagnostischen Mittels in Form eines Teststreifens kann ferner ein offener Film dienen, in dem neben Filmbildner und Pigmenten die spezifischen Antikörper bzw. -fragmente, die beschriebenen unspezifischen Antikörper(fragment)-Aggregate, ein geeignetes Puffersystem und sonstige üblicherweise für diagnostische Mittel verwendete Zusatzstoffe enthalten sind.

Die fertigen Testpapiere und Testfilme können als solche verwendet werden oder in an sich bekannter Weise an Trägerfolien angeklebt oder vorzugsweise zwischen Kunststoffen und feinmaschigen Netzwerken gemäß DE-PS 2118455 eingesiegelt werden und mit der zu untersuchenden Körperflüssigkeit (z. B. Blut, Plasma, Serum) in Verbindung gebracht werden.

Die Erfindung eignet sich zur Bestimmung aller Antigene mit wenigstens zwei Antigendeterminanten. Beispiele hierfür sind Thyreotropin (TSH), carcinoembryonales Antigen (CEA), Hepatitis-Viren (Hepatitis B surface antigen, HBs), 1-Fetoprotein (AFP), humanes Choriongonadotropin (HCG), luteinisierendes Hormon (LH), follikelstimulierendes Hormon (FSH), $\beta_2$-Microglobulin, saure Prostataphosphatase, Prolactin, Ferritin und Insulin.

Die folgenden Beispiele erläutern die Erfindung weiter:

**Beispiel 1**

**Herstellung von monoklonalem Maus-IgG-Aggregat durch Vernetzung mit einem homobifunktionellen Reagenz**

Monoklonales MAK33-IgG (> 95 % rein; Subklassenzusammensetzung K, $\gamma$ 1; Spezifität anti-Kreatinkinase) wird aus Ascitesflüssigkeit durch Ammoniumsulfatfällung und Chromatographie an DEAE-Ionenaustauscher isoliert (siehe A. Johnstone und R. Thorpe, Immunochemistry in practice, Blackwell Scientific Publications 1982, Seite 44 - 45).

50 mg IgG werden in 3 ml 0,025 M Bikarbonat/Karbonatpuffer pH 9,5 gelöst. In diese Lösung pipettiert man 17 $\mu$l 12,5%ige Glutardialdehydlösung und inkubiert 2 h bei 25° C. Anschließend wird die Lösung in einem Eisbad abgekühlt und mit 50 mM Triethanolaminpuffer ad pH 8,2 eingestellt. Dieser Lösung setzt man 0,6 ml frisch bereiteter Natriumborhydridlösung (8 mg Natriumborhydrid/1 ml bidest. Wasser) zu und inkubiert weitere 2,5 h bei 0° C. Durch 16 h Dialyse bei 0 - 4° C gegen 10 mM Phosphatpuffer pH 75, enthaltend 0,2 M NaCl, werden überschüssige Reagentien entfernt. Das Dialysat wird durch Ultrafiltration konzentriert auf ein Volumen von 1,25 ml. Ein Teil dieses Konzentrats (Rohgemisch) wird direkt zur Enstörung eingesetzt; 1,0 ml davon werden über eine Gelfiltrationssäule mit AcA22-Füllung (LKB) chromatographiert. Säulendimension 2 x 40 cm; Betriebspuffer 10 mM Phosphat/100 mM NaCl pH 7,5. Das Eluat der Säule wird mit einem UV-Monitor bei 280 nm auf Proteingehalt untersucht und fraktioniert. Die Fraktionen des gesamten proteinhaltigen Elutionsbereichs werden zu 4 Pools mit gleichem Volumen vereinigt. Durch Eichung der Gelchromatographiesäule mit Proteinen bekannten Molekulargewichts können den Pools die Molekulargewichtsbereiche 160 000 - 400 000, 400 000 - 1 000 000, 1 - 2 Mio und 2 - 10 Mio zugeordnet werden. Nach Konzentrierung der Pools auf eine Proteinkonzentration von ca. 2 mg/ml können die IgG-Aggregatlösungen der verschiedenen Molekulargewichtsbereiche zur Entstörung eingesetzt werden.

**Beispiel 2**

**Herstellung von monoklonalem Maus-IgG-Aggregat durch Vernetzung mit einem heterobifunktionellen Reagenz**

a) Herstellung von MAK33-IgG-MH (Maleinimidohexanoyl-MAK33-IgG).

100 mg MAK33-IgG werden in 4 ml 30 mM Phosphatpuffer pH 7,1 gelöst. Zu dieser Lösung pipettiert man 20 $\mu$l (4 $\mu$mol) einer 0,2 M Lösung von Maleinimidohexanoylsuccinimidat in Dimethylsulfoxid. Das Reaktionsgemisch wird 1 h bei 25° C inkubiert, dann 16 h gegen 10 mM Phosphatpuffer pH 6,1, enthaltend 50 mM NaCl, bei 0 - 4° C dialysiert. Man erhält 4,45 ml Lösung mit 22 mg MAK33-IgG-MH/ml.

b) Herstellung von MAK33-IgG-SAMS (S-Acetylmercaptosuccinyl-MAK33-IgG)

100 mg MAK33-IgG werden in 4 ml 0,1 M Phosphatpuffer pH 8,0 gelöst. Zu dieser Lösung pipettiert man 40 $\mu$l einer 0,25 M Lösung von S-Acetyl-mercaptobernsteinsäureanhydrid in Dimethylsulfoxid. Das Reaktionsgemisch wird 1 h bei 25° C inkubiert, dann 16 h gegen 10 mM Phosphatpuffer pH 6,1, enthaltend 50 mM NaCl, bei 0 - 4° C dialysiert. Man erhält 4,45 ml mit 21,,8 mg MAK33-IgG-SAMS/ml.

c) Vernetzung von MAK33-IgG-MH mit MAK33-IgG-MS (Mercaptosuccinyl-MAK33-IgG)

50 mg MAK33-IgG-SAMS verdünnen zu einer Konzentration von 15 mg/ml in 25 mM Phosphatpuffer pH 6,5, enthaltend 2 mM Ethylendiamintetraacetat (Puffer A). Zu dieser Lösung pipettiert man 75 $\mu$l einer 1 M Hydroxylaminlösung und inkubiert 20 min bei 25° C.

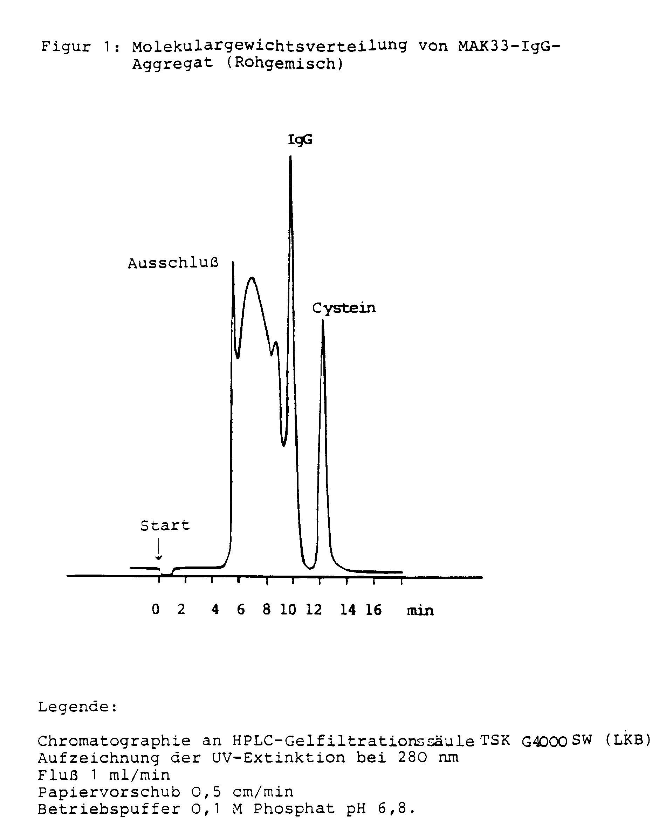

3 ml dieser Lösung mit 44 mg MAK33-IgG-MS werden mit Puffer A ad 6,0 ml verdünnt. Dieser Lösung setzt man 4 ml Lösung mit 88 mg MAK33-IgG-MH zu. Das Gemisch wird 40 min bei 25° C inkubiert, dann zum Abbruch der Vernetzungsreaktion ad 2 mM mit Cystein versetzt. Nach weiterer 30minütiger Inkubation bei 25° C wird ad 5 mM N-Methylmaleinimid zugegeben und weiter 1 h bei 25° C gehalten. Die Reaktionslösung wird gegen 10 mM Phosphatpuffer pH 7,5, enthaltend 0,2 M NaCl, dialysiert. Das Dialysat wird durch Ultrafiltration auf eine Proteinkonzentration von 35 mg/ml konzentriert und durch Zentrifugation von geringer Trübung befreit. Eine typische Molekulargewichtsverteilung für ein solches Dialysat (Rohgemisch) eines MAK33-IgG-Aggregats zeigt Fig. 1. Das Aggregatgemisch kann direkt oder nach Auftrennung in Molekulargewichtsfraktion (AcA22-Chromatographie wie in Beispiel 1) zur Entstörung eingesetzt werden.

**Beispiel 3**

**Herstellung von MAK33-IgG-Aggregat durch Vernetzung über Aminodextran**

a) Herstellung von S-Acetylthioacetyl-Aminodextran

100 mg Aminodextran (Dextran T40, Pharmacia®, 28 Aminogruppen/Molekulargewicht 40.000) werden in 4 ml 30 mM Phosphatpuffer pH 7,1 gelöst. Zu dieser Lösung pipettiert man 0,25 ml einer 0,2 M S-Acetylthioacetylsuccinimidat-Lösung in Dimethylsulfoxid. Das Reaktionsgemisch wird 1 h bei 25° C inkubiert, dann gegen 10 mM Phosphatpuffer pH 6,1, enthaltend 50 mM NaCl, dialysiert. Ausbeute 90 mg S-Acetylthioacetylaminodextran in 4,5 ml Lösung.

b) Vernetzung von MAK33-IgG-MH mit Thioacetyl-Aminodextran

2 ml Lösung mit 20 mg/ml S-Acetylthioacetyl-Aminodextran werden vorsichtig mit 0,1 M NaOH auf pH 6,5 eingestellt und Ethylendiamintetraacetat ad 2 mM zugesetzt. Zu dieser Lösung pipettiert man 40 $\mu$l einer 1 M Hydroxylaminlösung und inkubiert 20 min bei 25° C. Anschließend wird die Lösung mit 25 mM Phosphatpuffer pH 6,5 verdünnt auf 6,8 ml und mit 7,2 ml einer Lösung mit 158,4 mg MAK33-IgG-MH (Herstellung wie Beispiel 2a) versetzt. Nach 30 min Inkubation bei 25° C gibt man zum Reaktionsgemisch Cystein ad 2 mM und nach 30 min zusatzlich N-Methylmaleinimid ad 5mM hinzu. Nach einer weiteren Stunde Inkubation bei 25°C wird das Polymerisat diaylsiert gegen 10 mM Phosphatpuffer pH 7,5/50 mM NaCl. Nach Abzentrifugieren einer schwachen Trübung erhält man 19,5 ml einer Lösung von MAK33-IgG-Dextran-Aggregat mit einem Proteingehalt von 7,1 mg/ml.

**Beispiel 4**

**Herstellung von MAK33-IgG-Aggregat durch Vernetzung über Rinder-IgG**

a) Herstellung von Rinder-IgG-MH

100 mg polyklonales Rinder-IgG werden analog zu Beispiel 2a) mit 4 $\mu$mol Maleinimidohexanoylsuccini-midat umgesetzt. Ausbeute 4,45 ml Lösung mit 22 mg Rinder-IgG-MH/ml.

b) Herstellung von MAK33-SATA (S-Acetylthioacetyl-MAK33-IgG)

100 mg MAK33-IgG werden in 4 ml 30 mM Phosphatpuffer pH 7,1 gelöst. Zu dieser Lösung pipettiert man 20 $\mu$l (2$\mu$mol) einer 0,1 M Lösung von S-Acetylthioacetylsuccinimidt in Dimethylsulfoxid. Das Reaktionsgemisch wird 1 h bei 25 °C inkubiert, dann 16 h bei 0 - 4 °C gegen 10 mM Phosphatpuffer pH 6,1/50 mM NaCl dialysiert. Man erhalt 4,45 ml Lösung mit 22 mg MAK33-IgG-SATA/ml.

c) Vernetzung von Thioacetyl-MAK33-IgG mit Rinder-IgG-MH

50 mg MAK33-IgG-SATA werden mit 25 mM Phosphatpuffer pH 6,5, enthaltend 2 mM Ethylendiamintetraacetat (Puffer A) auf eine Konzentration von 15 mg/ml verdünnt. Zu dieser Lösung pipettiert man 75 $\mu$l einer 1 M Hydroxylaminlösung und inkubiert 20 min bei 25 °C.
3 ml dieser Lösung mit 44 mg Thioacetyl-MAK33-IgG setzt man 4 ml Lösung mit 88 mg Rinder-IgG-MH zu und inkubiert 25 min bei 25 °C. Zum Abbruch der Vernetzung wird sodann Cystein ad 2 mM und nach 30 min Inkubation bei 25 °C Jodacetamid ad 5 mM zugegeben. Nach einer weiteren Stunde Inkubation bei 25 °C wird 16 h bei 0 - 4 °C gegen 10 mM Phosphatpuffer pH 7,5/50 mM NaCl dialysiert. Nach Zentrifugation des Dialysats erhält man 8,9 ml MAK33-IgG-Rinder-IgG-Aggregat mit einer Proteinkonzentration von 13 mg/ml.

**Beispiel 5**

**Herstellung von MAK33-IgG-Aggregat vernetzt über MAK33-Fab**

a) Herstellung von MAK33-Fab-SATA

200 mg MAK33-IgG werden mit Papain zu Fab/Fc gespalten und MAK33-Fab aus dem Gemisch durch Chromatographie an DE52 Cellulose (Whatman) isoliert (Methode siehe A. Johnstone und R. Thorpe, Immunochemistry in Practice, Blackwell Scientific Publications 1982, 52 - 53). Ausbeute 95 mg MAK33-Fab als salzfreies Lyophilisat.

50 mg MAK33-Fab werden in 2 ml 30 mM Phosphatpuffer pH 7,1 gelöst. Zu dieser Lösung pipettiert man 30 $\mu$l (3 $\mu$mol) einer 0,1 M Lösung von S-Acetylthioacetylsuccinimidat in Dimethylsulfoxyd. Das Reaktionsgemisch wird 1 h bei 25 °C inkubiert, dann 16 h bei 0 - 4 °C gegen 10 mM Phosphatpuffer pH 6,1/50 mM NaCl/2 mM Ethylendiamintetraacetat dialysiert. Man erhält 2,6 ml Lösung mit 18,5 mg MAK33-Fab-SATA/ml.

b) Vernetzung von MAK33-IgG-MH mit Thioacetyl-MAK33-Fab

30 mg MAK33-Fab-SATA werden mit 25 mM Phosphatpuffer pH 6,5 auf eine Konzentration von 15 mg/ml verdünnt. Zu dieser Lösung pipettiert man 50 $\mu$l einer 1 M Hydroxylaminlösung und inkubiert 20 min bei 25 °C.

1,5 ml dieser Lösung mit 22 mg Thioacetyl-MAK33-Fab werden mit 55 mg MAK33-IgG-MH (hergestellt wie 2a) versetzt und mit bidest. Wasser auf ein Gesamtvolumen von 10 ml verdünnt. Der Ansatz wird 35 min bei 25 °C inkubiert und dann zum Abbruch der Vernetzung mit Cystein ad 2 mM gebracht. Nach 30 min bei 25 °C wird N-Methylmaleinimid ad 5 mM zugegeben und erneut 1 h bei 25 °C inkubiert. Anschließend wird die Reaktionslösung 16 h bei 0 - 4 °C gegen 10 mM Phosphatpuffer pH 7,5/0,1 M NaCl dialysiert. Nach Zentrifugation erhält man 12,8 ml klare Lösung mit 5,3 mg MAK33-IgG-Fab-Aggregat/ml.

**Beispiel 6**

**Herstellung von MAK33-IgG-Aggregat durch Vernetzung über Biotin/Streptavidin**

a) Herstellung von biotinyliertem MAK33-IgG.

50 mg MAK33-IgG in 2.5 ml 0.1 M Kaliumphosphatpuffer, pH 8.5 wurden mit 1.13 mg Biotinyl-$\epsilon$-aminocapronsäure-N-hydroxysuccinimid (Boehringer Mannheim GmbH in 50 $\mu$l Dimethylsulfoxid versetzt (Molverhältnis IgG : Biotin = 1 : 7.5) und nach Mischen 90 min. bei 25 °C inkubiert. Das biotinylierte IgG wurde über Nacht bei 4 °C gegen 2 mM Kaliumphosphatpuffer, pH 7.0 dialysiert.
Ausbeute: 45 mg MAK33-IgG-Biotin in 6.4 ml.

b) Vernetzung mit Streptavidin.

4 ml der MAK33-IgG-Biotin-Lösung wurden im Abstand von 5 min mit 3 Anteilen von je 1.5 mg Streptavidin (Boehringer Mannheim GmbH) in 50 mM Kaliumphosphatpuffer/0.1 M Natriumchlorid versetzt und 20 min bei 25 °C inkubiert. Molverhältnis IgG : Streptavidin = 2.5 : 1. Der Ansatz wurde durch Ultrafiltration auf 2 ml konzentriert und wie in Beispiel 1 an einer AcA22 Säule chromatographiert. Alle Fraktionen mit Molekulargewicht > 320 000 wurden vereinigt.
Ausbeute: 21 mg MAK33-IgG-Streptavidin Aggregat in 12 ml Lösung mit 17 mg IgG-Gehalt.

**Beispiel 7**

**Vernetzung von MAK-anti-Human-Albumin mit Human-Albumin**

40 mg Human-Albumin (Behringwerke, Marburg) in 1 ml 50 mM Kaliumphosphatpuffer, pH 7.0 wurden durch 3 h Erwärmen auf 70 °C aggregiert. Nach Abkühlen auf 25 °C wurde ein Anteil mit 25 mg Human-Albumin-Aggregat mit 50 mM Kaliumphosphatpuffer/0.1 M NaCl, pH 7.0 ad 2.5 mg/ml verdünnt und in Abständen von 5 min mit 4 Anteilen von je 2.5 mg MAK MI-anti-Human-Albumin (Gamma 1, Kappa) in 0.2 ml 50 mM Kaliumphosphatpuffer, pH 7.0 versetzt. Nach 20 min Inkubation bei 25 °C wurde durch Ultrafiltration auf 2 ml konzentriert und wie oben an einer AcA Gel-Säule chromatographiert. Die Protein-haltigen Fraktionen mit Molekulargewicht > 320 000 wurden vereinigt.
Ausbeute: 12.4 mg MAK MI-IgG-Albumin-Aggregat in 7.8 ml mit 8.86 mg IgG Gehalt.

**Beispiel 8**

**Vergleich der Entstörung von nativem MAK33-IgG und verschiedenen MAK33-IgG-Aggregaten in einem CEA Enzym-Immunoassay mit zwei spezifischen MAK Reaktanten**

Es wurden die Reagentien aus einer Testpackung Enzymun[R] CEA (Boehringer Mannheim GmbH) verwendet. Die in diesem Test enthaltenen Reagenzröhrchen sind mit einem monoklonalen, CEA-spezifischen Maus-IgG (IgG 1, K) beschichtet. Der Enzymmarkierte Reaktant ist ein monoklonales, CEA-spez. Maus-Fab-Peroxidase Konjugat; Subklassenzusammensetzung des Fab ist K, $\gamma$1).

Die verschiedenen MAK33-IgG-Präparate wurden in steigender Konzentration jeweils dem Inkubationspuffer zugefügt und der Test mit einem Humanserum (P43) durchgeführt, in dem auffallend effektive Störfaktoren nachgewiesen waren. Tabelle 1 zeigt die Konzentration MAK33-IgG-Präparat (in $\mu$g Protein/ml) im Inkubationspuffer, welche die Störung soweit unterdrückte, daß der richtige Analytgehalt im Normalbereich (1-3,5 ng/ml) gefunden wurde.

Tabelle 1

| Relative Entstörfunktion verschiedener MAK33-IgG-Präparate | | | | |
|---|---|---|---|---|
| Zusatz zum Inkubationspuffer | $\mu$g/ml | Entstörfaktor | Extinktion | scheinbarer CEA-Gehalt in Serum P43 ng/ml |
| ohne | 0 | - | 1.636 | > 58* |
| MAK33-IgG monomer | 9600 | 0.0026 | 0.132 | 3.4 |
| MAK33-IgG-Aggregat/GDA (Rohgemisch 1) | 34 | 0.74 | 0.126 | 3.1 |
| MAK33-IgG-Aggregat (Rohgemisch 2c) | 25 | 1 | 0.125 | 3.1 |
| MAK33-IgG-Dextran (3) | 43 | 0.58 | 0.129 | 3.3 |
| MAK33-IgG-Rinder-IgG-Aggregat (4) | 82 | 0.3 | 0.130 | 3.3 |
| MAK33-IgG-Fab-Aggregat (5) | 3 | 8.33 | 0.122 | 2.9 |

* Wert liegt außerhalb des durch die Eichpunkte definierten Bereiches der Eichkurve.

**Beispiel 9**

**Entstörwirkung von MAK-IgG-Aggregaten, die über nicht kovalente, bioaffine Bindungen vernetzt wurden, in einem CEA Enzymun Test.**

Reagentien und Testdurchführung siehe Beispiel 8.
Der tatsächliche CEA-Gehalt der verwendeten Humanserumprobe, bestimmt mit einer Referenzmethode, betrug 2.8 - 4.2 ng/ml. Tabelle 2 zeigt die an einer Eichkurve abgelesenen scheinbaren CEA-konzentrationen für eine Humanserumprobe (Nr. 108), welche Störfaktoren enthält, bei verschiedenen IgG-Aggregat-Zusätzen zum Inkubationspuffer.

Tabelle 2

| Entstörwirkung von IgG-Aggregaten mit bioaffiner Vernetzung | | |
|---|---|---|
| Zusatz zum Inkubationspuffer | $\mu$g IgG/ml | Humanserum Nr. 108 ng CEA/ml |
| ohne | - | 40 |
| MAK33-IgG monomer | 200 | 33.4 |
| | 500 | 31.4 |
| MAK33-IgG-Streptavidin Aggregat Beisp. 6 | 1 | 28.5 |
| | 10 | 8.8 |
| MAK MI-IgG-Hum.Albumin | 2.5 | 36.7 |
| Aggregat Beisp. 7 | 7.5 | 25.1 |

Aus der Tabelle ist ersichtlich, daß beide bioaffinvernetzten IgG-Aggregate auf IgG Gewichtsmenge bezogen wesentlich effektiver entstören als unvernetztes IgG: Faktor > 500 bzw. > 60. Die Einsatzmengen wurden so gewählt, daß sich nur teilweise Enstörung ergab; unter solchen Bedingungen kann nämlich die relative Entstöwirkung von verschiedenen Präparaten am besten abgeschätzt werden.

**Beispiel 10**

**Abhängigkeit der Entstörfunktion vom Molekulargewicht des MAK33-IgG-Aggregats**

Reagentien und Durchführung für diesen Versuch sind wie beschrieben im Beispiel 8. Die MAK33-IgG-Präparate sind hergestellt nach Beispiel 2.

## Tabelle 3

## Entstörwirkung von MAK33-IgG-Aggregaten in Abhängigkeit vom Molekulargewicht

| Zusatz zum Inkubationspuffer | Patientenserum Nr.18[1] Extinktion | ngCEA/ml[2] | Patientenserum Nr.43[1] Extinktion | ngCEA/ml[2] |
|---|---|---|---|---|
| ohne Zusatz | 1.567 | > 58[3] | 1.636 | > 58[3] |
| 125 µg/ml monomeres MAK33-IgG | 0.148 | 3.9 | 1.703 | > 58 |
| 10 µg/ml MAK33-IgG-Aggregat Rohgemisch | 0.115 | 2.7 | 0.179 | 6.2 |
| 30 µg/ml MAK33-IgG-Aggregat Rohgemisch | – | – | 0.125 | 3.1 |
| 5 µg/ml MAK33-IgG-Aggregat MG 2-10 Mio | 0.105 | 2.1 | 0.137 | 3.9 |
| 5 µg/ml MAK33-IgG-Aggregat MG 1-2 Mio | 0.109 | 2.4 | 0.285 | 11.0 |
| 5 µg/ml MAK33-IgG-Aggregat MG 400.000-1 Mio | 0.155 | 4.8 | 1.051 | 53.7 |
| 5 µg/ml MAK33-IgG-Aggregat MG 160.000-400.000 | 0.364 | 16.3 | 1.239 | > 58 |

1  Der mit einer Referenzmethode ermittelte wahre Gehalt an CEA liegt im Bereich 1 - 3,5 ng/ml.

2  Scheinbarer CEA-Gehalt ermittelt über Extinktion und Eichkurve mit CEA-Standardproben nach Arbeitsanleitung für Enzymun CEA.

3  Außerhalb des durch Eichpunkte definierten Eichkurvenbereichs.

**Beispiel 11**

**Vergleich der Entstörwirkung von MAK-IgG-Aggregaten, hergestellt aus verschiedenen monoklonalen Antikörpern**

Es wurden Teströhrchen aus einer Enzymun® TSH-Packung (Boehringer Mannheim GmbH) verwendet, die mit einem monoklonalen Anti-TSH Maus-IgG(IgGl, K) beschichtet sind. Die übrigen Reagentien wurden wie im Beispiel 8 der Enzymun CEA-Packung entnommen. Testführung nach Arbeitsanleitung dieser Packung. Bei dieser Testführung kann es kein Signal durch einen Analytgehalt einer Serumprobe geben, weil die beiden spezifischen Reaktanten unterschiedliche Spezifität haben. Es handelt sich somit nur um einen Modell-Test, der mit Serumproben nur dann Signal über dem Leerwert ergibt, wenn Störfaktoren enthalten sind. Die zum Vergleich herangezogenen MAK-IgG-Aggregate wurden analog Beispiel 2 c aus MAK33 hergestellt.

Tabelle 4

| Vergleich von IgG-Aggregaten verschiedener monoklonaler Antikörper in der Entstörwirkung bei einem Modell-Test | | |
|---|---|---|
| Zustatz zum Inkubationspuffer | $\mu$g/ml | Extinktion bei Serum P43 |
| MAK33-IgG-Aggregat (2c) Rohgemisch | 0 | 0.900 |
|  | 1.25 | 0.506 |
|  | 2.5 | 0.366 |
|  | 5 | 0.238 |
|  | 10 | 0.195 |
|  | 20 | 0.152 |
| MAK MS43.10-IgG-Aggregat Rohgemisch | 0 | 0.900 |
|  | 1.25 | 0.554 |
|  | 2.5 | 0.406 |
|  | 5 | 0.286 |
|  | 10 | 0.202 |
|  | 20 | 0.180 |

Extinktion für eine Serumprobe ohne CEA und ohne Störfaktor: 0.162

Ergebnis: Die Entstörwirkung verschiedener monoklonaler MAK-IgG-Aggregate ist im Rahmen der Fehlergrenzen gleich.

**Beispiel 12**

**Entstörwirkung von MAK33-IgG-Aggregat und monomerem MAK33-IgG in einem Test mit biotinylierten MAK-IgG-Reaktanten**

11

In diesem Test werden zwei monoklonale Heptatitis-Oberflächenantigen(HBsAg)-spezifische MAK-IgGs in biotinylierter Form eingesetzt (Biotinylierung durchgeführt nach T.V. Updyke und G.L. Nicolson in Methods in Enzymology, 121 (1986) 717-725). Der eine davon, MAK6E7-IgG ist vom Subtyp IgGl/K, der andere, MAK5A10-IgG, ist vom Subtyp IgG2a, K. Als Enzymmarkierter Reaktant wurde MAK5A10-Fab-POD-Konjugat verwendet.

Zusammensetzung des Inkubationspuffers:

40 mM Phosphatpuffer pH 7,0
0,2 M Natriumtartrat
0,5 % (w/v) Rinderserumalbumin
0,1 % (w/v) Rinder-IgG
0,5 % (w/v) Pluronic® F68
0,01 % (w/v) Phenol
200 ng/ml MAK6E7-IgG (biotyniliert)
25 ng/ml MAK5A10-IgG (biotyniliert)
200 mU/ml MAK5A10-Fab-POD-Konjugat
Entstörproteinzusatz siehe Tabelle 5.
MAK33-IgG-Aggregat Rohgemische waren hergestellt nach Beispiel 2c.
Für den Test wurden je 0,2 ml Serumprobe und 1 ml Inkubationspuffer in ein Polystyrolröhrchen, das mit Streptavidin beschichtet war, pipettiert. Dann wurde 4 h bei RT inkubiert. Anschließend wurde jedes Röhrchen mit 3 x 1,5 ml Leitungswasser gewaschen und je 1 ml Substratlösung (ABTS/Peroxyd) aus Enzymun CEA-Testpackung zugegeben. Nach einer weiteren Stunde Inkubation bei RT wurde die Extinktion in der umgesetzten Substratlösung bei 405 nm gemessen.

Tabelle 5

| Vergleich der Entstörwirkung von MAK33-IgG-Präparaten in einem Sandwich-Enzymimmun-Test mit biotinylierten MAK-IgG-Reaktanten | | | | |
|---|---|---|---|---|
| Zusatz zum Inkubationspuffer | $\mu$g/ml | Extinktion für Serumprobe | | |
| | | Nr. 100[1] | Nr. 489[1] | NS[2] |
| ohne | 0 | 1.390 | 0.262 | 0.042 |
| MAK33-IgG monomer | 1 | - | - | 0.042 |
| | 5 | 1.070 | 0.102 | 0.045 |
| | 10 | 0.884 | 0.068 | 0.048 |
| | 200 | 0.125 | 0.042 | 0.046 |
| | 400 | 0.080 | 0.048 | 0.049 |
| MAK33-IgG-Aggregat (2c) Rohgemisch | 0 | 1.044 | 0.378 | 0.047 |
| | 1 | 0.125 | 0.034 | 0.043 |
| | 5 | 0.043 | 0.045 | 0.048 |
| | 10 | 0.048 | 0.035 | 0.054 |

ad 1: Die Serumproben Nr. 100 und 489 wurden von einer Blutbank bezogen und waren als HBsAg frei deklariert.
ad 2: Serum eines gesunden Spenders, das weder HBsAg noch Störfaktor enthielt.

Aus dem Befund, daß für Serum Nr. 100 dieselbe Enstörung auf ein Signal von 0,125 erreicht wird mit 200 $\mu$g/ml monomerem MAK33-IgG oder 1 $\mu$g/ml MAK33-IgG-Aggregat folgt, daß für diesen Test IgG-Aggregat 200fach entstör-aktiver ist.

**Beispiel 13**

**Entstörwirkung von MAK33-IgG-Aggregat in einem Test mit Trockenchemie-Reagenzträger**

Die Bestimmung wird mit Trockenchemiereagenzträger in einem Einschrittest nach dem Doppel-Antikörper Festphase-Sandwich-Prinzip in Rotoreinsatzelementen mit einem Zentrifugalanalysator mit der in der DE-OS 3425 008.5 beschriebenen Analyseapparatur durchgeführt.

1. Herstellung der Reagenzlösungen

a) Puffer I

50 mmol/l Kaliumphosphat-Puffer, pH 6,0 hergestellt durch Mischung von 50 mmol/l $K_2HPO_4$-Lösung und 50 mmol $KH_2PO_4$-Lösung bis zum Erreichen des pH-Wertes 6,0.

b) Puffer II

Puffer II wird wie Puffer I hergestellt mit dem Unterschied, daß als pH-Wert der pH 7,5 eingestellt wird und daß der Puffer zusätzlich 10 g/l Rinderserumalbumin und 150 mmol/l NaCl enthält.

c) Reaktant $R_1$-Lösung, bindefähig mit TSH

Als Reaktant $R_1$ wird ein monoklonaler Maus-anti-TSH-Antikörper eingesetzt. Die diesen Antikörper enthaltende Ascitesflüssigkeit wird ad 1,8 M mit Ammoniumsulfat versetzt. Das Präzipitat wird in einem Puffer aus 15 mM Natriumphosphat, pH 7,0 und 50 mM Natriumchlorid aufgenommen. Die so erhaltene Lösung wird einer Passage über DEAE-Cellulose unterworfen. Der so erhaltene, TSH-bindefähige Antikörper wird biotinyliert (5 Biotin/IgG) und mit Puffer II auf eine Protein-Konzentration von 1 mg/ml verdünnt.

d) Enzymmarkierte Reaktant $R_2$-Lösung

Als Reaktant $R_2$ wird ebenfalls ein monoklonaler Maus-Anti-TSH-Antikörper eingesetzt, der jedoch eine andere antigene Determinante als Reaktant $R_1$ erkennt. Die diesen Antikörper enthaltende Ascitesflüssigkeit wird, wie unter 1.3 angegeben, aufgereinigt. Der vollständige Antikörper wird in bekannter Weise nach der Methode von R.R. Porter, Biochem. J. 73, (1959), S. 119, zum Fab-Fragment gespalten. Die erhaltenen Fab-Fragmente werden gemäß Ishikawa et al., J. of Immunoassay 4 (1983), S. 209 - 327, mit $\beta$-Galactosidase gekoppelt. Die Reaktant $R_2$-Lösung wird in Puffer II auf eine Konzentration von 500 mU/ml (gemessen mit o-Nitrophenyl-$\beta$-Galactosid bei 37°C) verdünnt.

e) Entstörprotein

MAK33-IgG-Polymer, hergestellt wie in Beispiel 2, wird in Puffer II auf eine Konzentration von 1 mg/ml gelöst.

f) Avidin-Lösung

Avidin wird in Puffer I auf eine Proteinkonzentration von 50 ug/ml verdünnt.

g) Substratlösung

| | |
|---|---|
| Chlorphenolrot-$\beta$-galactosid (hergestellt nach DE-OS 3345748) | 5 mmol/l (3,05 g/l) |
| HEPES | 70 mmol/l (16,7 g/l) |
| NaCl | 154 mmol/l (9 g/l) |
| Rinderserumalbumin | 0,3 % (3 g/l) |
| Tween® 20 | 0,2 % (2 g/l) |
| pH (mit NaOH) | 7,25 |

2. Herstellung von Reagenzträgern

a) Reagenzträger 1 (ohne Entstörprotein)

40 $\mu$l einer Lösung, die pro Liter 100 mmol Natriumphosphat pH 7,3 (37°C), 2 mmol Magnesiumchlorid, 9 g Natriumchlorid, 5 g Rinderserumalbumin, 0,5 mg biotinylierten Anti-TSH monoklonalen Antikörper aus Maus (Reaktant $R_1$), 1 000 U Anti-TSH-Antikörper-(Maus)-Fab-Fragment-$\beta$-Galactosidase-Konjugat (Reaktant $R_2$-Lösung) (Aktivität bestimmt mit ortho-Nitrophenyl-$\beta$-D-galactosid bei 37°C) enthält, wird auf ein Vlies aufgetropft, das aus kommerziellem Polyesterpapier besteht. Anschließend wird bei Raumtemperatur getrocknet. Diese Vliese werden bis zu ihrer Verwendung bei 4°C und einer relativen Luftfeuchtigkeit von 20 % aufbewahrt.

b) Reagenzträger 1′ (mit Entstörprotein)

Die Herstellung erfolgt wie für Reagenzträger 1 bis auf den Unterschied, daß zusätzlich pro Liter Tränklösung 0,01 g MAK 33-IgG-Aggregat aus Beispiel 2 c (Rohgemisch) enthalten sind.

c) Reagenzträger 2

Auf ein Zellulosevlies wird nach dem Bromcyan-Aktivierungsverfahren (DE-OS 1768512) Avidin (Avidin-Lösung) fixiert, wobei pro Gramm Fasermaterial 10 ug Avidin zur Fixierung angeboten werden. Durch Waschen wird ungekoppelter Antikörper entfernt und das Vlies schonend bei Raumtemperatur getrocknet. Die Lagerung der so erhaltenen Vliese folgt analog Reagenzträger 1.

3. Durchführung der Bestimmung

Die Bestimmung mit Hilfe dieser beiden Reagenzträger 1 und 2, bzw. 1′ und 2, erfolgt mit der in der DE-OS 3425008.5 beschriebenen Vorrichtung zur Durchführung analytischer Bestimmungen.

Diese lehrt ein Rotoreinsatzelement für Zentrifugalanalyseautomaten, bestehend aus einem Formkörper, der eine Probenauftragskammer, die mit einer Mehrzahl von Reagenzfeldern in Verbindung steht, die jeweils ein mit einem bestimmten Reagenz imprägniertes saugfähiges Trägermaterial enthalten, wenigstens eine Mischventilkammer und eine Meßkammer aufweist, die zusammen einen Probenflüssigkeitstransportweg bilden, der von radial innen nach radial außen führt, wenn das Einsatzelement auf dem Rotor befestigt ist, und weiter wenigstens eine weitere Kammer zur Meßführung führt und mit dem Probenflüssigkeitstransportweg mindestens teilweise identisch ist, aufweist.

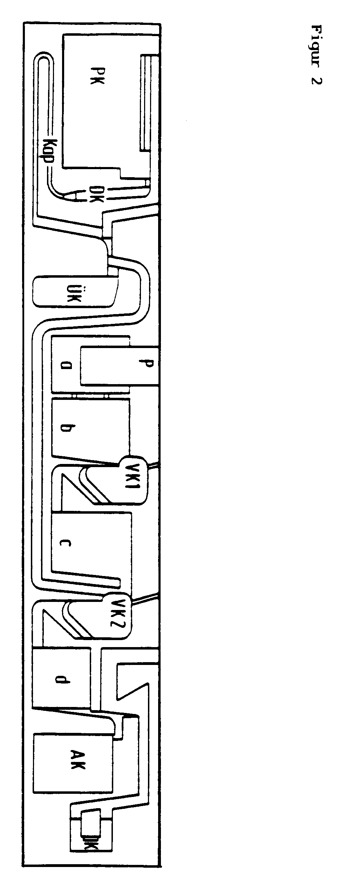

Dabei führt der Probenflüssigkeitstransportweg von einer Probenauftragskammer (P) über eine mit saugfähigem Material gefüllte, Puffer enthaltende Kammer (a), eine Kammer (b) und eine zwischen den Kammern (a) und (c) angeordnete erste Ventilkammer (VK1) zu einer zweiten Ventilkammer (VK2) und von dieser über die Kammer (d) und über eine Auffangkammer (AK) zur Meßkammer (K). Zur Aufnahme einer weiteren Flüssigkeit ist eine als Pumpenkammer ausgebildete Substratkammer (PK) vorgesehen, welche über eine Dosierkammer (DK) und Kapillare (Kap) bestehende Dosiereinrichtung und eine Überlaufkammer (ÜK) mit der zweiten Ventilkammer (VK2) verbunden ist (siehe Figur 2).

Zur Bestimmung der Extinktion a (Meßsignal inklusive Störsignal) werden Reagenzträger 1 und Reagenzträger 2 benutzt, zur Bestimmung der Extinktion b (spezifisches Meßsignal ohne Störsignal) werden Reagenzträger 1′ und 2 benutzt.

Reagenzträger 1 bzw. 1′ wird auf Feld c des Rotoreinsatzelementes plaziert und Reagenzträger 2 auf Feld d. Dabei werden 40 $\mu$l konzentrierter Probe durch eine Öffnung am oberen Rand direkt auf das Feld a pipettiert. 270 $\mu$l Substratlösung werden in Kammer PK pipettiert. Durch ein geeignetes Zentrifugationsprogramm, bei dem hohe Drehzahlen mit Stillstand abwechseln, werden dann Probe und Substratlösung in Richtung Trennmatrix und Küvette gefördert.

Dabei werden im Verlauf des Programms die Reaktanten $R_1$ und $R_2$ ohne bzw. mit Entstörprotein durch die Probenflüssigkeit vom Feld c eluiert und die homogene Mischung anschließend zur Reaktion gebracht. Auf Feld d werden die gebildeten Komplexe über $R_1$ an Avidin gebunden. Der Transfer der Probe von Feld c nach d erfolgt innerhalb sehr kurzer Zeit.

Die Substratlösung wird durch die Dosierkammer DK in Portionen geteilt, von denen die ersten zum Auswaschen von überschüssigem, nicht komplexiertem Konjugat dienen.

Störfaktoren, die $R_2$ vernetzen könnten, werden bei Einsatz von 10 $\mu$g Entstörprotein/ml Testlösung neutralisiert und ebenfalls ausgewaschen (Einsatz von Reagenzträger 1′).

Die über Komplexbildung auf d gebundene $\beta$-Galactosidase-Aktivität ist proportional zur in der Probe enthaltenen TSH-Menge bzw. zum Probenleerwert. Diese Aktivität wird mit einer weiteren Substratportion

14

bestimmt, wobei das Substrat in einer 5minütigen Reaktion zu farbigen Produkten umgesetzt wird. Die gebildete Farbe und die weitere Farbenentwicklung/min in der flüssigen Phase werden in der Küvette bei 576 nm gemessen.

Unter diesen Bedingungen wurden folgende Resultate erhalten:

Tabelle 6

| Probe | a | | b | |
|---|---|---|---|---|
| | Ext. [mE] | Konz. [$\mu$U/ml] | Ext. [mE] | Konz. [$\mu$U/ml] |
| TSH Kalibrator 0 $\mu$U/ml[c)] | 451 | 0 | 457 | 0 |
| TSH-Kalibrator 19,6 $\mu$U/ml[c)] | 3331 | 19,6 | 3311 | 19,5 |
| Humanserum 1 | 669 | 1,5 | 725 | 1,8 |
| Humanserum 43 mit Störfaktoren | 6790 | 38 | 662 | 1,5 |

Alle Messungen wurden bei $\lambda$ = 576 nm bei einer Schichtdicke von 0,3 cm durchgeführt und auf Schichtdicke d = 1 cm umgerechnet

a) TSH-Bestimmung bei Verwendung von Reagenzträger I

b) TSH-Bestimmung bei Verwendung von Reagenzträger 1' mit MAK33-IGG-Polymer zur Neutralisation von M-Fabspezifischen Störfaktoren

c) TSH-Standards kalibriert am 2. IRP 80/588.

Der Gehalt an TSH in Humanserum 43 wurde mit einem Enzymun® TSH Test der Fa. Boehringer Mannheim zu 1.4 $\mu$U/ml bestätigt. Dieser Test enthält als spezifische Reaktanten einen an Tube beschichteten MAK-anti-TSH und ein polyklonales Schaf-anti-TSH-Fab-POD-Konjugat. Letzteres ist inert gegen Störfaktoren in HS 43.

## Patentansprüche

1. Verfahren zur Bestimmung einer immunologisch bindefähigen Substanz in einer biologischen Flüssigkeit von einer ersten Tierart oder vom Menschen in Gegenwart von mindestens zwei für die immunologisch bindefähige Substanz spezifischen Reaktanten einer zweiten Tierart, von denen mindestens einer der Reaktanten $F_c$-haltig ist, und in Gegenwart eines Inhibitors zur Kompensation von Störfaktoren, dadurch gekennzeichnet, daß das Reaktionsgemisch mit 0,1 - 50 $\mu$g/ml für die immunologisch bindefähige Substanz nicht spezifischen, von monoklonalen oder polyklonalen Antikörpern abgeleiteten, nicht-partikulären Aggregaten mit einem Molekulargewicht von mindestens 320.000 Dalton einer zweiten und/oder dritten Tierart zur Kompensation von Störfaktoren in Berührung gebracht wird.

2. Verfahren gemäß Anspruch 1, dadurch gekennzeichnet, daß es sich bei der immunologisch bindefähigen Substanz um ein polyvalentes Antigen, bei den spezifischen Reaktanten um Antikörper, von denen mindestens einer ein Fab- bzw. F(ab')$_2$-Fragment ist, und bei den nicht spezifischen Aggregaten um aggregierte IgGs handelt.

3. Verfahren nach Anspruch 1 oder 2, dadurch gekennzeichnet, daß das Reaktionsgemisch mit 5 - 25 $\mu$g/ml nicht spezifischen IgG-Aggregaten in Berührung gebracht wird.

4. Verfahren nach Anspruch 1 oder 2, dadurch gekennzeichnet, daß das Reaktionsgemisch mit 5 - 10 $\mu$g/ml nicht spezifischen IgG-Aggregaten in Berührung gebracht wird.

5. Verfahren nach einem der Ansprüche 2 bis 4, dadurch gekennzeichnet, daß die nicht spezifischen IgG-Aggregate aus IgG und einem zweiten Makromolekül bestehen.

6. Verfahren nach Anspruch 5, dadurch gekennzeichnet, daß es sich bei dem zweiten Makromolekül um nicht spezifische IgGs einer dritten Tierart, um nicht spezifische Fc-freie IgG-Fragmente, Proteine, Polysaccharide oder ein anderes wasserlösliches Polymer handelt.

7. Verfahren nach einem der Ansprüche 1 bis 6, dadurch gekennzeichnet, daß die nicht spezifischen Aggregate ein Molekulargewicht von 320.000 bis 10 Millionen Dalton aufweisen.

**8.** Verfahren nach einem der Ansprüche 1 bis 7, dadurch gekennzeichnet, daß die nicht spezifischen Aggregate durch Hitzeeinwirkung, chemische oder nicht-kovalente Vernetzung gebildet werden.

**9.** Verfahren nach einem der Ansprüche 1 bis 8, wobei mindestens einer der spezifischen Reaktanten mit einer Markierungssubstanz verbunden und ein anderer an eine Matrix fixiert oder biotinyliert ist, und entweder die fixierte bzw. an ein Biotin-bindendes Protein assoziierte immunologisch gebundene oder die immunologisch nicht-gebundene Aktivität bestimmt wird, dadurch gekennzeichnet, daß die nicht spezifischen IgG-Aggregate in Lösung in dem Reaktionsgemisch während des Assays vorhanden sind.

**10.** Diagnostisches Mittel zur Bestimmung einer immunologisch bindefähigen Substanz in einer biologischen Flüssigkeit von einer ersten Tierart oder vom Menschen, enthaltend mindestens zwei für die immunologisch bindefähige Substanz spezifische Reaktanten einer zweiten Tierart, von denen mindestens einer der Reaktanden Fc-haltig ist, einen nicht-partikulären Inhibitor zur Kompensation von Störfaktoren, eine geeignete Puffersubstanz sowie gegebenenfalls weitere üblicherweise verwendete Hilfsstoffe, dadurch gekennzeichnet, daß der Inhibitor für die immunologisch bindefähige Substanz nicht spezifische, von monoklonalen oder polyklonalen Antikörpern abgeleitete Aggregate einer zweiten und/oder dritten Tierart mit Molekulargewichten über 320.000 Dalton ist.

**11.** Diagnostisches Mittel nach Anspruch 10, dadurch gekennzeichnet, daß es sich bei der immunologisch bindefähigen Substanz um ein polyvalentes Antigen, bei den spezifischen Reaktanten um Antikörper, von denen mindestens einer ein Fab- bzw. F(ab')$_2$-Fragment ist, und bei den nicht spezifischen Aggregaten um aggregierte IgGs handelt.

**12.** Diagnostisches Mittel nach Anspruch 10 oder 11, dadurch gekennzeichnet, daß als nicht spezifische IgG-Aggregate aus IgG und einem zweiten Makromolekül bestehende IgG-Aggregate verwendet werden.

**13.** Diagnostisches Mittel nach Anspruch 12, dadurch gekennzeichnet, daß es sich bei dem zweiten Makromolekül um nicht spezifische IgGs einer dritten Tierart, um nicht spezifische Fc-freie IgG-Fragmente, Proteine, Polysaccharide oder ein anderes wasserlösliches Polymer handelt.

**14.** Diagnostisches Mittel nach einem der Ansprüche 10 bis 13, dadurch gekennzeichnet, daß zwei oder mehrere spezifische Maus-oder Ratten-MAK-Reaktanten, von denen mindestens ein Reaktant ein Fab- oder F(ab')$_2$-Fragment ist, und homopolymere oder heteropolymere nicht spezifische IgG-Aggregate oder IgG/Fab- bzw. F(ab')$_2$-Aggregate mit Molekulargewichten größer als 320.000 Dalton zugesetzt werden, wobei die in den unspezifischen Aggregaten enthaltenen IgGs und Fab-bzw. F(ab')$_2$-Fragmente monoklonal und von der Spezies und Subklasse sind wie mindestens einer der spezifischen Reaktanten.

**15.** Diagnostisches Mittel nach einem der Ansprüche 10 bis 14, dadurch gekennzeichnet, daß die nicht spezifischen IgG-Aggregate ein Molekulargewicht von 320.000 bis 10 Millionen Dalton aufweisen.

**16.** Diagnostisches Mittel nach Anspruch 10 bis 15, dadurch gekennzeichnet, daß als zusätzliche Hilfsmittel Reaktionsbeschleuniger, Detergentien und/oder Stabilisatoren verwendet werden.

**Claims**

**1.** Process for the determination of an immunologically-bindable substance in a biological liquid from a first animal species or from humans in the presence of at least two reactants of a second animal species specific for the immunologically-bindable substance, of which at least one of the reactants is F$_c$-containing, and in the presence of an inhibitor for the compensation of disturbing factors, characterised in that, for the compensation of disturbing factors, the reaction mixture is brought into contact with 0.1 - 50 μg/ml of non-particular aggregates, not specific for the immunologically-bindable substance, derived from monoclonal or polyclonal antibodies, with a molecular weight of at least 320,000 Dalton, of a second and/or third animal species.

**2.** Process according to claim 1, characterised in that in the case of the immunologically-bindable substance it is a question of a polyvalent antigen, in the case of the specific reactants of antibodies of

16

EP 0 331 068 B1

which at least one is a Fab or F(ab')$_2$ fragment and in the case of the non-specific aggregates of aggregated IgGs.

3. Process according to claim 1 or 2, characterised in that the reaction mixture is brought into contact with 5 - 25 $\mu$g/ml of non-specific IgG aggregates.

4. Process according to claim 1 or 2, characterised in that the reaction mixture is brought into contact with 5 - 10 $\mu$g/ml of non-specific IgG aggregates.

5. Process according to one of claims 2 to 4, characterised in that the non-specific IgG aggregates consist of IgG and a second macromolecule.

6. Process according to claim 5, characterised in that in the case of the second macromolecule it is a question of non-specific IgGs of a third animal species, of non-specific Fc-free IgG fragments, proteins, polysaccharides or another water-soluble polymer.

7. Process according to one of claims 1 to 6, characterised in that the non-specific aggregates have a molecular weight of 320,000 to 10 million Dalton.

8. Process according to one of claims 1 to 7, characterised in that the non-specific aggregates are formed by the action of heat, chemical or non-covalent cross-linking.

9. Process according to one of claims 1 to 8, whereby at least one of the specific reactants is bound with a labelling substance and another is fixed on to a matrix or biotinylated and either the fixed activity or activity associatedly immunologically bound on a biotin-binding protein or the immunologically non-bound activity is determined, characterised in that the non-specific IgG aggregates are present in solution in the reaction mixture during the assay.

10. Diagnostic agent for the determination of an immunologically-bindable substance in a biological liquid of a first animal species or of humans, containing at least two reactants of a second animal species specific for the immunologically bindable substance, of which at least one of the reactants is $F_c$-containing, a non-particular inhibitor for the compensation of disturbing factors, a suitable buffer substance, as well as possibly further adjuvant materials usually employed, characterised in that the inhibitor for the immunologically-bindable substance is non-specific aggregates of a second and/or third animal species with molecular weights above 320,000 Dalton derived from monoclonal or polyclonal antibodies.

11. Diagnostic agent according to claim 10, characterised in that in the case of the immunologically-bindable substance it is a question of a polyvalent antigen, in the case of the specific reactants of antibodies, of which at least one is a Fab or F(ab')$_2$ fragment, and in the case of the non-specific aggregates of aggregated IgGs.

12. Diagnostic agent according to claim 10 or 11, characterised in that, as non-specific IgG aggregates, IgG aggregates are used consisting of IgG and a second macromolecule.

13. Diagnostic agent according to claim 12, characterised in that in the case of the second macromolecule it is a question of non-specific IgGs of a third animal species, of non-specific $F_c$-free IgG fragments, proteins, polysaccharides or another water-soluble polymer.

14. Diagnostic agent according to one of claims 10 to 13, characterised in that two or more specific mouse or rat MAB reactants, of which at least one reactant is a Fab or F(ab')$_2$ fragment, and homopolymeric or heteropolymeric non-specific IgG aggregates or IgG/Fab or F(ab')$_2$ aggregates with molecular weights greater than 320,000 Dalton are added thereto, whereby the IgGs and Fab or F(ab')$_2$ fragments contained in the non-specific aggregates are monoclonal and of the species and sub-class like at least one of the specific reactants.

15. Diagnostic agent according to one of claims 10 to 14, characterised in that the non-specific IgG aggregates have a molecular weight of 320,000 to 10 million Dalton.

17

**16.** Diagnostic agent according to claim 10 to 15, characterised in that reaction accelerators, detergents and/or stabilisers are employed as additional adjuvants.

**Revendications**

**1.** Procédé pour la détermination d'une substance capable d'effectuer une liaison immunologique, dans un fluide biologique d'une première espèce animale ou d'homme, en présence d'au moins deux réactifs spécifiques de la substance capable d'effectuer une liaison immunologique, d'une seconde espèce animale, dont au moins l'un des réactifs contient le $F_c$, et en présence d'un inhibiteur pour la compensation de facteurs perturbateurs, caractérisé en ce que le mélange réactionnel est mis en contact avec 0,1-50 $\mu$g/ml d'un agrégat non particulaire dérivant d'anticorps monoclonaux ou polyclonaux, non spécifique de la substance capable d'effectuer une liaison immunologique, ayant une masse moléculaire d'au moins 320.000 daltons, d'une seconde et/ou troisième espèce animale, pour compenser des facteurs perturbateurs.

**2.** Procédé selon la revendication 1, caractérisé en ce que la substance capable d'effectuer une liaison immunologique est un antigène polyvalent, le réactif spécifique est un anticorps, l'un au moins étant un fragment Fab ou F(ab'), et l'agrégat non spécifique est de l'IgG agrégé.

**3.** Procédé selon la revendication 1 ou 2, caractérisé en ce que le mélange réactionnel est mis en contact avec 5-25 $\mu$g/ml d'agrégat d'IgG non spécifique.

**4.** Procédé selon la revendication 1 ou 2, caractérisé en ce que le mélange réactionnel est mis en contact avec 5-10 $\mu$g/ml d'agrégat d'IgG non spécifique.

**5.** Procédé selon l'une quelconque des revendications 2 à 4, caractérisé en ce que l'agrégat d'IgG non spécifique est constitué d'IgG et d'une deuxième macromolécule.

**6.** Procédé selon la revendication 5, caractérisé en ce que la deuxième macromolécule est de l'IgG non spécifique d'une troisième espèce animale, un fragment IgG exempt de Fc non spécifique, une protéine, un polysaccharide ou un autre polymère hydrosoluble.

**7.** Procédé selon l'une quelconque des revendications 1 à 6, caractérisé en ce que l'agrégat non spécifique a une masse moléculaire de 320.000 à 10 millions de daltons.

**8.** Procédé selon l'une quelconque des revendications 1 à 7, caractérisé en ce que l'agrégat non spécifique a été formé par réticulation chimique ou non covalente, sous l'effet de la chaleur.

**9.** Procédé selon l'une quelconque des revendications 1 à 8, au moins l'un des réactifs spécifiques étant lié à une substance de marquage et un autre étant fixé sur une matrice ou étant biotinylé, et l'on détermine soit l'activité liée immunologiquement, associée à une protéine liant la biotine ou fixée, soit l'activité non liée immunologiquement, caractérisé en ce que l'agrégat d'IgG non spécifique est présent en solution dans le mélange réactionnel pendant le dosage.

**10.** Agent de diagnostic pour la détermination d'une substance capable d'effectuer une liaison immunologique dans un fluide biologique d'une première espèce animale ou d'homme, contenant au moins deux réactifs spécifiques de la substance capable d'effectuer une liaison immunologique, d'une seconde espèce animale, au moins l'un des réactifs contenant du Fc, un inhibiteur non particulaire pour la compensation des facteurs perturbateurs, une substance tampon appropriée ainsi qu'éventuellement d'autres adjuvants usuels, caractérisé en ce que l'inhibiteur de l'agrégat dérivant d'anticorps monoclonaux ou polyclonaux, non spécifiques de la substance capable d'effectuer une liaison immunologique, d'une seconde et/ou troisième espèce d'animal, présente une masse moléculaire supérieure à 320.000 daltons.

**11.** Agent de diagnostic selon la revendication 10, caractérisé en ce que la substance capable d'effectuer une liaison immunologique est un antigène polyvalent, le réactif spécifique est un anticorps, au moins l'un étant un fragment Fab ou F(ab')$_2$, et l'agrégat non spécifique est de l'IgG agrégé.

18

**12.** Agent de diagnostic selon la revendication 10 ou 11, caractérisé en ce que comme agrégat d'IgG non spécifique, on utilise de l'agrégat d'IgG constitué d'IgG et d'une seconde macromolécule.

**13.** Agent de diagnostic selon la revendication 12, caractérisé en ce que la seconde macromolécule est de l'IgG non spécifique d'une troisième espèce animale, un fragment IgG exempt de Fc non spécifique, une protéine, un polysaccharide ou un autre polymère hydrosoluble.

**14.** Agent de diagnostic selon l'une quelconque des revendications 10 à 13, caractérisé en ce que deux ou plusieurs réactifs MAK spécifiques de souris ou de rats, au moins l'un des réactifs étant un fragment Fab ou F(ab')$_2$, et un agrégat d'IgG non spécifique, homopolymère ou hétéropolymère, ou un agrégat IgG Fab ou F(ab')$_2$, ayant une masse moléculaire supérieure à 320.000 daltons, est ajouté, et l'IgG ainsi que les fragments Fab ou F(ab')$_2$ contenus dans l'agrégat non spécifique sont monoclonaux et appartiennent à l'espèce ou à la subdivision à laquelle appartient au moins l'un des réactifs spécifiques.

**15.** Agent de diagnostic selon l'une quelconque des revendications 10 à 14, caractérisé en ce que l'agrégat d'IgG non spécifique a une masse moléculaire de 320.000 à 10 millions de daltons.

**16.** Agent de diagnostic selon les revendications 10 à 15, caractérisé en ce que, comme adjuvant supplémentaire, on utilise des accélérateurs de réaction, des détergents et/ou des stabilisants.

Figur 1: Molekulargewichtsverteilung von MAK33-IgG-
Aggregat (Rohgemisch)

Legende:

Chromatographie an HPLC-Gelfiltrationssäule TSK G4000 SW (LKB)
Aufzeichnung der UV-Extinktion bei 280 nm
Fluß 1 ml/min
Papiervorschub 0,5 cm/min
Betriebspuffer 0,1 M Phosphat pH 6,8.

EP 0 331 068 B1

Figur 2